# EUROPEAN PATENT APPLICATION

(11) **EP 2 865 373 A1**
(43) Date of publication of application: **29.04.2015**
(21) Application number: 14190242.9
(22) Date of filing: 24.10.2014
(51) Int. Cl.: A61K 9/48, A61K 9/70, A23L 1/00

(54) **Compounding and extruding method for producing starch softgel capsules**

(30) Priority: 26.10.2013 CN 201310510572
(71) Applicant: Zhongshan Capsule Starch Material Technology Co., Ltd., Guangdong 528451 (CN)
(72) Inventor: Shuai, Fangwen, 410331 Changsha (CN); Wang, Xiangfeng, 410331 Changsha (CN); Zhang, Jiawei, 410331 Changsha (CN); Zhang, Nuozi, 410331 Changsha (CN)
(74) Representative: Clark, Jane Anne

(57) **Abstract**

Described as a method for preparing starch softgel capsules, which, specifically, involves in the following three steps: 1, Extruding starch premix and gel solution into films respectively; 2, Forming composite starch film through co-extrusion method; 3, Processing the composite starch film into starch softgel capsules.

## Description

### CROSS REFERENCE TO RELATED APPLICATION

This application claims priority from Chinese Patent Application No. 201310510572.X, filed with State Intellectual Property Office, P. R. C. on October 26, 2013, the entire content of which is incorporated herein by reference.

### FIELD OF INVENTION

This invention relates to a preparation method of softgel capsules, more specifically, of non-gelatin softgel capsules.

### BACKGROUND OF THE INVENTION

Softgel capsules are generally used for enclosing medicines that should not directly contact esophagus and that must be disintegrated in the stomach or intestine. At present, most capsules used for medicine or dietary supplements are made of gelatin extracted from animal skins or bones, and the gelatin extracting process involves acid or alkali treatment, which unavoidably generates animal protein residues. These residues will interact with the enclosed content and thus leads to negative consequences such as drug spoilage.

In addition, animal-derived gelatin is not acceptable to vegetarians and/or the people with Islamic and Jewish beliefs, and those with allergic constitution should also avoid intake of gelatin products.

As starch is made from rich source of plants, its price is far lower than that of gelatin. The starch, when modified, achieves better gelation performance and mechanical strength, making its characteristics close to that of gelatin. Since starch softgel capsules will overcome the shortcomings of existing gelatin capsules and at the same time, has the advantage of low cost, It is worthy of popularization.

Conventionally, the preparation methods of most starch softgel capsules are as follows: feed the prepared starch gel into the softgel capsule machine, and then use a mold or glue for molding. In the conventional preparation of softgel capsules, the glue is coated on the mold to form thin grooved slices, which are then encapsulated into softgel capsules after the processes of material feeding and roller pressing. Though this is a method easy and convenient to operate, the softgel capsules such made has poor encapsulation performance and uneven coating thickness, which affects the product quality.

An embodiment of the present invention to provide a preparation method of starch softgel capsules, which includes the following steps:

(1) Preparation of composite starch film: Mix starch, anti-caking agent and gelatinization to get starch-based premix and mix gel, water retention agent, and emulsifier in water to form paste liquid. Then, process the said premix into starch-based extrusion film and the said paste liquid into gel extrusion film through extrusion mechanism respectively. Apply co-extrusion method to merge these two types of film into a composite film with the following specification: thickness, 0.2-1mm; Tensile Modulus, 3 MPa; Elongation at break, 80%-140%.

(2) Preparation of starch softgel capsules: Process two pieces of composite starch films into starch softgel capsules using rotary die method.

### Description of Each Step

### 1. Preparation of composite starch films

Composite starch films are prepared with starch and gel by complex method.

Said complex method as follows:
Step A: Mix the starch and anti-caking agent for 2 minutes in a high-speed mixer at the speed of 1500-2000rpm and under the temperature of 60°C, and then slowly add gelatinization into the high-speed mixer while keep stirring for another 5 minutes.
Step B: Feed the starch-based premix into a double screw-type extruder to make starch granules. Then process the granules into starch-based extrusion films using single screw-type extruder.
Step C: Mix gel, water retention agent, and emulsifier in water to form paste liquid, which is then fed into a single-screw extruder to be processed into gel extrusion film.
Step D: Place the gel extrusion film on the upper roller and the starch-based extrusion film on the lower one. Then merge these two types of film into one composite film and extrude it out through the co-extrusion die head.

The starch mentioned in the above step A is native starch or modified type by chemical or physical processes, preferably, esterified cassava starch.

The anti-caking agent mentioned in the above step A is pharmaceutical stearic acid or fatty glyceride

The gelatinization mentioned in the above step A is deionized water.

The parameters of the double screw-type extruder mentioned in the above step B are as follows: material temperature, 25-170 °C; screw RPM, 60-400 rpm; (Optimized speed should be: 60-160 °C, 100-150 °C, 70 °C, 80 °C, 90 °C, 100 °C, 120 °C, 130 °C, 140 °C)

The gel mentioned in the above step C is one or a combination of the substances selected from the group consisting of amylopectin, gellan gum, carrageenan, xanthan gum, and carrageenan.

The emulsifier mentioned in the above step C is an ionic emulsifier, preferably pharmaceutical alkali metal salts sulfate or alkali metal salts sulfonate, such as pharmaceutical sodium dodecyl sulfonate or pharmaceutical sodium dodecyl sulfate.

The water retention agent mentioned in the above step C is pharmaceutical glycerin or pharmaceutical grade sorbitol.

The parameters of the single screw-type extruder mentioned in the above step C are as follows: material temperature, 25-110 °C; screw rotation speed, 60-300 rpm; (Optimized speed should be: 50-160 °C, 100-150 °C, 70 °C, 80 °C, 90 °C, 100 °C, 120 °C, 130 °C, 140 °C),

The per-unit-area weight ratio of the gel extrusion film and the starch-based extrusion film (which are used to form composite starch film) according to above step D ranges from 1:10 to 1:20.

The thickness of the composite starch film mentioned in the above step D is 0.2-1mm; the tensile modulus, greater than 3MPa; and the elongation at break, 80%-140%.

### (2) Preparation of starch softgel capsules

Starch softgel capsules are prepared by rotary die process, which is described as follows:

Feed two pieces of composite starch films into two closely adjoining cylinder moulds of the rotary die machine, with the gel-attached side facing upwards; adjust the mould temperature to 40-90 °C and start up the machine. The two cylinder moulds drive the two composite films spinning inward in different direction. The grooves on the mould can be vacuumed to make the composite starch films adhered to the mould form pits, which are then pressed by the mould into softgel capsules with cavities, while at the same time of the softgel capsule formation, the softgel capsule content is filled from right above the junction of the two cylinder moulds.

One thing that needs to be pointed out here is that the gel extrusion film and the starch-based extrusion film are not of simply overlying double-layer structure, so the starch softgel capsules prepared using this invention has its superiority in terms of stability and encapsulation.

The starch softgel capsules prepared according to this invention can be used to prepare drugs, dietary supplements, and functional foods.

### EXAMPLES

The examples set forth below further explain the content of this present invention and the nature of the products manufactured by this invention. The filled content of the softgel capsules in all the examples is 250mg cod-liver oil, and the following examples here are illustrative, and should not be viewed as limiting the scope of the present invention

### Example 1

The raw materials used to prepare starch-based extrusion film and their weight ratio:
Starch: corn starch, 85%;
Anti-caking agent: pharmaceutical stearic acid, 1.2%;
Gelatinization: Deionized water, 13.8%.

The raw materials used to prepare gel extrusion film and their weight ratio:
Gel: carrageenan, 70%;
Water retaining agent: pharmaceutical glycerin, 20%;
Emulsifier: pharmaceutical sodium dodecyl sulfate, 2%;
Deionized water: 8%.

### Compounding Steps:

(a) Mix the starch and anti-caking agent for 2 minutes in the high-speed mixer at the speed of 1500-2000RPM and under the temperature of 60 °C, then slowly add gelatinization into the high-speed mixer while keep stirring for another 5 minutes to get the starch-based premix ready.
(b) Feed the starch-based premix into the double-screw extruder, and then extrude the mixed material into starch granules, which are made into starch-based extrusion film using single screw-type extruder.
(c) Dissolve gel, water retention agent , and emulsifier in water to form gel solution, which is then fed into single screw extruder to form gel extrusion film.
(d) Place the gel extrusion film on the upper roller and the starch-based extrusion film on the lower one to extrude composite starch film through co-extrusion die head

Starch softgel capsules preparation: Put the composite film into the rotary die with temperature control device to make starch softgel capsules. The mold temperature is set at 60 °C.

The parameters of the double-screw extruder according to above step (b) are as follows

The starch-based film is extruded out at the speed of 350 RPM and the designed temperature for each slider is as follows:
Slide 1: 25 °C
Slide 2-3: 110 °C
Slide 4-6: 145 °C
Slide 7-9: 165 °C
Slide 10-12: 160 °C
Nozzle: 160 °C

The parameters of the single screw-type extruder according to above step (b) are as follows:

The gel extrusion film is extruded at the speed of 350 RPM, and the designed temperature is 160 °C.

The parameters of the single screw-type extruder according to above step (c) are as follows:

The gel extrusion film is extruded at the speed of 300 RPM, and the designed temperature is 105 °C.

The per-unit-area weight ratio of the gel extrusion film and the starch-based extrusion film that are used to form composite starch film is 1:15, and the thickness of the composite starch film is 0.5mm.

### Example 2

The raw materials used to prepare starch-based extrusion film and their weight ratio:
Starch: mung bean starch, 85%;
Anti-caking agent: pharmaceutical grade stearic acid, 1.2%;
Gelatinization: Deionized water, 13.8%

The raw materials used to prepare gel extrusion film material and their weight ratio:
Gel: carrageenan, 70%;
Water retaining agent: pharmaceutical glycerin, 20%;
Emulsifier: pharmaceutical sodium dodecyl sulfate, 2%;
Deionized water: 8%.

The per-unit-area weight ratio of the gel extrusion film and the starch-based extrusion film which is used to form composite starch film is 1:14.7, and the thickness of the composite starch film is 0.5mm. The processes are the same as those in Example 1

### Example 3

The raw materials used to prepare starch-based extrusion film and their weight ratio:
Starch: cassava starch, 85%;
Anti-caking agent: pharmaceutical grade stearic acid, 1.2%;
Gelatinization: Deionized water, 13.8%.

The raw materials used to prepare gel extrusion film and their weight ratio:
Gel: cross-linked cassava glue, 70%;
Water retaining agent: pharmaceutical glycerin, 20%;
Emulsifier: pharmaceutical sodium dodecyl sulfate, 2%;
Deionized water: 8%

The per-unit-area weight ratio of the gel extrusion film and the starch-based extrusion film which are used to form composite starch film is 1:16, and the thickness of the composite starch film is 0.5mm. The processes are the same as Example 1

### Example 4

The raw materials used to prepare starch-based extrusion film and their weight ratio:
Starch: esterified cassava starch, 85%;
Anti-caking agent: pharmaceutical grade stearic acid, 1.2%;
Gelatinization: Deionized water, 13.8%.

The raw materials used to prepare gel extrusion film and their weight ratio:
Gel: xanthan gum, 75%;
Water retaining agent: pharmaceutical glycerin, 10%;
Emulsifier: pharmaceutical sodium dodecyl sulfate, 4%;
Deionized water: 11%.

The per-unit-area weight ratio of the gel extrusion film and the starch-based extrusion film which are used to form composite starch film is 1:16.2, and the thickness of the composite starch film is 0.52mm. The processes are the same as those in Example 1.

### Example 5

The raw materials used to prepare starch-based extrusion film and their weight ratio:
Starch: cassava starch, 80%;
Anti-caking agent: pharmaceutical grade stearic acid, 3.2%;
Gelatinization: Deionized water, 16.8%.

The raw materials used to prepare gel extrusion film material and their weight ratio:
Gel: Cross-linked cassava starch, 85%;
Water retaining agent: pharmaceutical Sorbitol, 8%;
Emulsifier: pharmaceutical sodium dodecyl sulfate, 3%;
Deionized water: 4%

The per-unit-area weight ratio of the gel extrusion film and the starch-based extrusion film which are used to form composite starch film is 1:17, and the thickness of the composite starch film is 0.47mm. The processes are the same as those in Example 1

### Example 6

The raw materials used to prepare starch-based extrusion film and their weight ratio:
Starch: corn starch, 85%;
Anti-caking agent: pharmaceutical grade stearic acid, 1.2%;
Gelatinization: Deionized water, 13.8%.

The raw materials used to prepare gel extrusion film material and their weight ratio:
Gel: Carrageenan, 70%;
Water retaining agent: pharmaceutical glycerin, 20%;
Emulsifier: pharmaceutical sodium dodecyl sulfate, 2%;
Deionized water: 8%.

The processes are the same as Example 1.

The per-unit-area weight ratio of the gel extrusion film and the starch-based extrusion film which are used to form composite starch film is 1:13, and the thickness of the composite starch film is 0.5mm.

### Comparative Example 1

The raw material and weight ratio
Gel: guar gum, 2%;
Starch: corn starch, 80%;
Water retaining agent: pharmaceutical glycerin, 1%;
Anti-caking agent: pharmaceutical stearic acid, 0.2%;
Emulsifier: pharmaceutical sodium dodecyl sulfate, 0.02%;
Gelatinization: Deionized water, 16.78%.

Put the above materials into a jacked kettle, and then add the same amount of deionized water (by weight). Heat the water to 60°C and stir till it is fully dissolved and swelling; Keep the temperature for about 1 to 2 hours till the gel solution is ready. Prepare the softgel capsules according to the conventional preparation method of capsules.

### Comparative Example 2

The raw material and weight ratio
Starch: corn starch, 85%;
Anti-caking agent: pharmaceutical stearic acid, 1.2%;
Gelatinization: Deionized water, 13.8%.
Raw material used in paste liquid and weight ratio:
Gel: guar gum, 70%;
Water retaining agent: pharmaceutical glycerin, 20%;
Emulsifier: pharmaceutical sodium dodecyl sulfate, 2%;
Deionized water: 8%.

The following parameters of the double screw-type extruder:

Extruding out at the speed of 350 RPM and the designed temperature of each slider are as follows
Slide 1: 25 °C
Slide 2-3: 110 °C
Slide 4-6: 145 °C
Slide 7-9: 165 °C
Slide 10-12: 160 °C
Nozzle: 160 °C

Parameters of the single-screw extruder are as follows:
Screw RPM: 350 R / Min; Designed temperature: 150 °C

The steps are as follows:
(a) Mix the starch and anti-caking agent for 2 minutes in the high-speed mixer at the speed of 1500-2000rpm and under the temperature of 60 °C, then slowly add gelatinization into the high speed mixer machine while keep on stirring for another 5 minutes to form the starch-based premix.
(b) Feed the starch-based premix into the double-screw extruder to make starch granules; Then use single screw-type extruder to process the starch granules into starch-based extrusion film.
(c) Mix gel, water retention agent , and emulsifier in deionized water to form gel solution, which is then coated on the inner side of the starch-based extrusion film to form composite starch-based film.

Starch-based capsule preparation: Put the composite film into the rotary die with temperature control device to make starch-based capsules. The mold temperature is set at 60 °C.

Comparison of the elongation at break and the tensile modulus of the sample films taken form Example 1 to Example 6. (Refer to Table 1 for the result)

Take sample capsules prepared in each of the above 8 examples, and inspect the weight variation of the capsules from each example. The inspection method is as follow: (refer to Table 1 for the result)

Weight variation test: follow the method stated in the appendix I E of the Chinese Pharmacopoeia 2010 edition, in which, the weight variation limit is ±10%. Apply conventional approach to inspect the elongation at break and the tensile modulus under the same condition

According to the above examples, the softgel capsul prepared using this present invention displays excellent performance and complies with the standards of the Pharmacopoeia. It is superior to the softgel capsule made using existing conventional technology.

**Table 1:**

| Performance parameters of softgel capsules prepared using different method | | | |
|---|---|---|---|
| | Composite Starch Film | | Softgel Capsule |
| | tensile modulus (MPa) | Elongation at break (%) | Weight variation (%) |
| Example 1 | 5.8 | 120 | 2.25 |
| Example 2 | 5.4 | 110 | 2.14 |
| Example 3 | 5.1 | 115 | 2.24 |
| Example 4 | 8.0 | 200 | 1.09 |
| Example 5 | 5.4 | 130 | 2.12 |
| Example 6 | 5.3 | 100 | 2.65 |
| Comparative example | 2.5 | 60 | 6.74 |
| Comparative example | 3.5 | 80 | 5.32 |

An embodiment provides a method for preparing starch softgel capsules, which, specifically, involves in the following three steps: 1, Extruding starch premix and gel solution into films respectively; 2, Forming composite starch film through co-extrusion method; 3, Processing the composite starch film into starch softgel capsules.

## Claims

1. A method for preparing starch softgel capsules, comprising:
(1) preparation of composite starch film comprising:
mix starch, anti-caking agent and gelatinization to get starch-based premix and mix gel, water retention agent, and emulsifier in water to form paste liquid;
process the said premix into starch-based extrusion film and the said paste liquid into gel extrusion film by extrusion mechanism respectively, and use co-extrusion method to merge these two types of film into a composite film,
wherein the composite film has a thickness of 0.2-1mm, a tensile modulus of 3 MPa; and a elongation at break of 80%-140%; and
(2) preparation of starch softgel capsules comprising:
process the two composite starch films into starch softgel capsules by rotary die method.

2. The method according to claim 1, wherein the said gel is at least one selected from the group consisting of amylopectin, gellan gum, carrageenan, xanthan gum, and carrageenan.

3. The method according to claim 1 or 2, wherein the said starch is native starch or modified starch; wherein water retention agent is polyhydric fatty alcohol,; wherein anti-caking agent is pharmaceutical grade stearic acid or glycerol esters of fatty acids; and wherein emulsifier is ionic emulsifier.

4. The method according to claim 1, 2 or 3, wherein the said method for preparing starch-based extrusion film comprises: (a) Feed the starch-based premix into the double- screw extruder, through which, the premix is processed into starch granules; and (b) Process the starch granules into starch-based extrusion film through the single-screw extruder.

5. The method according to claim 1, 2 or 3, wherein the said double-screw extruder comprises a material temperature of 25-170°C and RPM of 60-400 R/M.

6. The method according to any preceding claim, wherein the said gel extrusion film is prepared by single screw extruder using air purge method, and the gel extrusion film comprises gel at 60%-90% by weight of the gel extrusion film; water retention agents at 0%-20% by weight of the gel extrusion film; and emulsifier at 0%-20% by weight of the gel extrusion film.

7. The method according to any preceding claim, wherein the starch-based extrusion film is placed on the lower roller while the gel extrusion film on the upper roller, and then the two films are merged into one composite film and extruded out through co-extrusion die head.

8. The method according to any preceding claim, wherein the per-unit-area weight ratio of the said gel extrusion film and starch-based extrusion film ranges from 1:10 to 1:20.

9. The method according to any preceding claim, wherein the said softgel capsules comprises:
DI water at 19-50%;
starch at 35-80%; Gel at 1-20%;
optionally, gelatinization maximum of 20%, Water retention agents maximum 5%, Emulsifier 0.05%, and anti-caking agent 0.5%; and
optionally, Pharmaceutical grade colorant.

10. The use of the starch softgel capsules in the fields of medicines, health products, and function foodsprepared using the method according to claim 9.
